# EUROPEAN PATENT APPLICATION

(11) **EP 4 147 713 A1**
(43) Date of publication of application: **15.03.2023**
(21) Application number: 21196406.9
(22) Date of filing: 13.09.2021
(51) Int. Cl.: A61K 39/00

(54) **A RNA VACCINE COMPRISING AN RNA POOL GENERATED FROM A DOUBLE-STRANDED DNA POOL**

(71) Applicant: OncoDNA, 6041 Gosselies (BE)
(72) Inventor: Detiffe, Jean-Pol, 6500 Beaumont (BE); Van Huffel, Christophe, 1332 Rixensart (BE)
(74) Representative: Calysta NV

(57) **Abstract**

A process for producing a RNA vaccine comprising a plurality of epitopes specifically deduced from a target comprising the steps of: obtaining a plurality of synthetic DNA constructs in pool encoding (i) a plurality of different epitopes deduced from the said target, and of transcribing in vitro the said plurality of synthetic DNAs into a corresponding plurality of RNAs, wherein the said target is a peptide from an infectious agent or cancer neoepitopes specifically identified in one patient and having an amino acid sequence different, by at least one amino acid, from the amino acid sequences naturally present in normal cells of the patient.

## Description

### Technical domain

The present disclosure concerns the use of information extracted from the genome of a patient to generate a ribonucleic acid RNA vaccine. In particular, the discovery of a process for producing a RNA vaccine comprising a plurality of epitopes specifically deduced from a target from an infectious agent or cancer neoepitopes.

More specifically, the present disclosure refers to a mRNA cancer vaccine for a patient having a tumor.

The present invention also refers to an ex *vivo* method to select neoantigens to the patient comprising the step of submitting a blood sample from the patient having been administered with the mRNA cancer vaccine.

In oncology, personalized vaccine, e.g. personalized therapeutic vaccine comprising tumor-specific antigens (neoantigens) of the tumor of the patient, to treat the tumor hold great promise as a next-generation of personalized cancer immunotherapy.

### State of the art

The concept of personalized cancer vaccination is based on the identification of the neoantigens able to trigger the appropriate immunological response. This will allow to generate an optimal personalized vaccine, which will specifically boost the patient's immune system in attacking the tumor.

RNA vaccines were developed in personalized cancer therapy but also to threat and/or the prevent different infectious diseases. The RNA vaccines may be used to induce a balanced immune response against cancers or against an infectious disease, comprising both cellular and humoral immunity, without risking the possibility of insertional mutagenesis, for example. The RNA vaccines may be utilized to treat and/or prevent a disease or a cancer of various stages or degrees of metastasis.

Currently, a critical limitation in RNA (mRNA) vaccine, which is especially true for personalized mRNA cancer vaccine, is to properly identify the specific epitopes, i.e. tumor neoepitopes, that will elicit a proper immunological response in the patient leading to a sufficient activation of T cells in the patient to attack efficiently the tumor and/or the disease.

The document EP3400005 relates to an anticancer vaccine being directed to a plurality of neoepitopes from tumor neoantigens. Thereby, a personalized neoantigen vaccine is obtained that specifically targets the identified tumor antigens. In one aspect, the present invention relates to a therapeutic anticancer vaccine comprising an immunologically effective amount of a polynucleotide comprising a nucleotide sequence encoding an antigenic unit comprising (i) from 2 to 50 antigenic subunits, each subunit comprising at least a part of a cancer neoepitope sequence and a linker, (ii) a final cancer neoepitope sequence.

Currently, the production of a mRNA vaccine is based on in vitro transcription of a DNA template. The DNA template, which can be a plasmid or can result from multiple ligation steps of a plurality of DNA fragments, generally encodes for concatemeric epitopes or neoepitopes.

Unfortunately, although the documents in the state of the art describe the merits of their technology, currently no method exists allowing the development of an mRNA vaccine triggering an immunological response in the patient with high efficiency. In mRNA cancer vaccine, only 4 to 20% of the neoepitopes encoded by the mRNA vaccine are able to activate a T cell response. A tumor can be characterized by numerous, e.g. more than 100, different neoepitopes. A critical challenge is thus to select the appropriate neoepitopes, or the epitopes of a specific disease, that will be incorporated into the mRNA vaccine to trigger a proper immunological response in the patient.

Current research developments try to improve the algorithms of prediction aiming to identify the 'best' neoantigen candidates, i.e. of a tumor of a patient, that will trigger an optimal immunological response in the patient characterized by activation of specific T cells of the patient. The present inventors do consider that the methods known in the state of the art to identify the best neoantigen candidates are based on affinity data between the neoantigen peptide and the HLA receptors available only for particular types of HLA receptors. Thus, the current methods to identify the best neoantigen candidates might reach some efficacy for patient having said particular types of HLA receptors, while the methods will be inefficient for a large proportion of patients having different particular types of HLA receptors.

### Summary of the invention

There is therefore a need to dispose of a process for producing a RNA vaccine comprising a plurality of epitopes specifically deduced from a target in the patient, wherein the plurality of epitopes has affinity for the specific HLA receptors of the patient.

The present invention provides a process for producing a RNA vaccine comprising a plurality of epitopes specifically deduced from a target comprising the steps of:
- obtaining a plurality of different synthetic DNA constructs in a pool encoding a plurality of different epitopes deduced from the said target being in proximity of a sequence allowing the translation of a RNA sequence into a peptide by a eukaryotic cell and having a promoter for the transcription into the said mRNA sequence of the said DNA construct encoding the said epitope and of the said sequence allowing the translation into a peptide,
and of
- transcribing in vitro the said plurality of synthetic DNAs into a corresponding plurality of RNAs,
wherein the said target is a peptide from an infectious agent, or cancer neoepitopes specifically identified in one patient as having at least one amino acid difference at the peptide level. In other words, this equates to peptides having at least one amino acid difference as compared with peptides from normal cells of the said patient. Indeed, the process according to the present invention, wherein the plurality of synthetic DNA constructs has been obtained in a pool, permits to have a high number, potentially an unlimited number, of different DNA constructs. As a consequence, the RNA vaccine according to the present invention can incorporate a very large number of epitopes of the infectious disease or of epitopes, i.e. neoepitopes, from a tumor and a number of them will lead to T cells activation of the patient.

According to the present invention, the immunological response in the patient refers to the production of specific T cells, preferably cytotoxic T cells, targeting the neoantigen(s) or the infectious agent. Alternatively, the immunological response is tested *in vitro* by measuring the production of one or more immunological markers, for example IFN-gamma, by T cells of the patient, wherein said T cells of the patient are put in contact with specific epitopes of the mRNA vaccine. Alternatively, or in addition, the immunological response is measured by the identification of CD8+ CD107a+ T cells from a blood sample of the patient having been administered with the mRNA vaccine of the present invention.

According to the present invention, the term "at least one amino acid difference as compared with peptides from normal cells" means amino acid sequences encoded from open reading frames present in the DNA or the RNA of a tumoral cell from a patient, or DNA or RNA from an infectious agent, and not present in normal cells, i.e. in peripheral blood mononuclear cell (PBMC), of the patient, or a matched, non cancerous, tissue.

The present invention also relates to a mRNA cancer vaccine for a patient having a tumor comprising a plurality of mRNAs encoding a plurality of neoantigens, wherein said plurality of neoantigens is deduced from the tumor, wherein said plurality of mRNAs encodes for at least 1% of all tumor neoantigens identified in the tumor, preferably for at least 2%, more preferably at least 5%, even more preferably at least 10%, 20%, 30%, 40%, 50%, 60%, 70%, 80%, 90% of all tumor neoantigens identified in the tumor.

The present invention also refers to a ex *vivo* method to select neoantigens to a patient comprising the step of submitting a blood sample from a patient having been administered with the mRNA cancer vaccine according to the present invention or with the RNA vaccine obtainable by the process according to the present invention to the corresponding synthetic peptide neoepitopes, or to one or several truncated version thereof, and of identifying which synthetic peptide neoepitopes triggers an immune response.

Other characteristics and advantages of the present invention will be derived from the non-limitative following description, and by making reference to the drawings and the examples.

According to the present invention, the term "neoantigen" refers to a tumor-specific antigen of the patient. In the framework of the present invention, a "neoantigen" comprises an amino acid sequence different, by at least one amino acid, as compared with peptides naturally present in normal cells of the patient.

According to the present invention, the term "neoepitope" refers to a tumor-specific peptide originating from a neoantigen able to bind to major histocompatibility complex (MHC) of the patient.

According to the present invention, the term "amino acid sequence different, by at least one amino acid, as compared with peptides naturally present in normal cells of the patient" means (i) a peptide having an amino acid sequence having at least one amino acid different at a corresponding position of the amino acid sequence naturally present in normal cells of the patient, and/or (ii) a peptide that is not present in the normal cells of the patient, i.e. a de novo amino acid sequence present in the tumor and not present in normal cells of the patient (this situation shares more similarities with vaccination with an amino acid sequence of an infectious agent), and is thus advantageous. In addition, this term "amino acid sequence different, by at least one amino acid, as compared with peptides naturally present in normal cells of the patient" covers neoantigens generated after translocation events or mutation in splicing sites; these events produce a peptide having a segment of high homology with a peptide from normal tissue and a C-terminal segment being different, or having a high homology with another peptide from a normal tissue. In other words, any genetic event producing a peptide not 100% identical to a peptide found in a normal tissue corresponds to the above definition.

In the present invention (process for generating the mRNA, the (starting) DNA construct, the obtained mRNA or the mRNA as such), preferably, the DNA, and thus the mRNA comprises a 3' Poly A segment. Preferably this 3' Poly A segment consists of 100% of adenosine, or consists essentially of adenosine; for instance stretches of at least 10 consecutive adenosine residues, preferably at least 20, 30, 40 consecutive adenosine residues, separated by other nucleotides (a stretch of less than 20, preferably less than 10 residues not forming the above stretch), and followed by a second stretch of at least 10 consecutive adenosine residues (independently of the length of the first stretch, at least 20, 30, 40 consecutive adenosine residues), possibly followed by separating nucleotides (less than 20, preferably less than 10 residues not forming a stretch) and subsequent adenosine stretches is also comprised by the terminology 'Poly A segment', provided that at least 90 adenosine residues are present, preferably at least 120 adenosine residues are present in these stretches, to form the 'Poly A segment'.

The inventors have developed a process for producing a mRNA vaccine comprising a plurality of epitopes specifically deduced from a target. This allows to ensure the administration of epitopes that will be recognized by the patient's immune system. The inventors have shown that such an approach provides a more robust immune response than a vaccination-based method where one, or a few, different epitope(s) have been administered to a patient, even if the epitopes are selected and/or designed using the state-of the art technology, including the bio-informatics tool developed to increase the predictability of an immune response. Such a positive result is surprising in view of the dilution of the potent epitopes, by the large amount of the non-potent ones. This process comprises the steps of :
- obtaining a plurality of different synthetic DNA constructs in a pool encoding a plurality of different epitopes deduced from the said target, being in proximity of a sequence allowing the translation of a RNA sequence into a peptide by a eukaryotic cell and having a promoter for the transcription of the said DNA construct encoding the said epitope and of the said sequence allowing the translation into a peptide,
and of
- transcribing *in vitro* the said plurality of synthetic DNAs into a corresponding plurality of RNAs,
wherein the said target is a peptide from an infectious agent or cancer neoepitopes specifically identified in one patient and having an amino acid sequence different, by at least one amino acid, as compared with peptides naturally present in normal cells of the patient.

Preferably, the transcription of the DNA into the RNA sequence is performed in the presence of pseudouridine and/or of N1-Methylpseudouridine, 15-methyluridine (m5U), and/or 2-thiouridine (s2U).

Preferably, the process to obtain the RNA sequence comprises a further step of adding a 5' Cap element, comprising two nucleotides separated (linked in 5'-3') by a triphosphate stretch. The two nucleotides can be natural, or modified, for instance by methylation of the -OH residue at the 2' position (2-O-Me nucleotides) in one or in the two residues forming the 5' Cap element. The 5' Cap element can comprise additional groups, including additional nucleotides, provided that two nucleotides are separated (linked in 5'-3') by a triphosphate group, instead of a monophosphate.

Preferably, the DNA construct (hence the encoded mRNA as well) further comprises a plurality of sequences elements encoding a peptide sequence for endosomial targeting of the encoded peptide. For example, the peptide sequence for endosomial targeting is the signal sequence of a lysosome-associated membrane protein (LAMP1 or LAMP2), for instance of dendritic cells (DC-LAMP) and/or a sequence targeting the epitope to the MHC-I receptor, such as SEQ ID NO:3.

Advantageously, the DNA construct (hence the encoded mRNA as well) further comprises a plurality of sequences elements encoding a peptide sequence for the loading of the encoded peptide for presentation to T lymphocytes. More preferably, the plurality of sequence elements encoding a peptide sequence for the loading of the encoded peptide for presentation to T lymphocytes is a dendritic cell lysosomal associated membrane glycoprotein signal sequence, such as SEQ ID NO:5.

Preferably, the plurality of the different DNA constructs share the same plurality of sequences elements encoding a peptide sequence for endosomial targeting of the encoded peptide and/or the same plurality of sequences elements encoding a peptide sequence for the loading of the encoded peptide for presentation to T lymphocytes.

Preferably, the sequence allowing the translation of a RNA sequence into a peptide by an eukaryotic cell is a translation enhancer/promoter sequence located in a 5'UTR region (upstream of a coding region) of the DNA construct. More preferably, the sequence allowing the translation is a translation enhancer/promoter sequence chosen from the group of translation enhancer/promoter sequence comprising Human Cytomegalovirus (CMV) enhancer, alpha or beta-globin enhancer (such as SEQ ID NO:2), EF-1 alpha enhancer, simian virus 40 (SV40) enhancer, PGK1 promoter, Ubiquitin C promoter, HSD17B4 and beta-Actin promoter.

Preferably, the DNA construct (hence the encoded mRNA as well) further comprises at least one sequence element encoding 3'-UTR and/or a 3' Poly A tail segment(s). More preferably, the 3' Poly A tail segment(s) is of at least 68 nucleotides, favorably at least 100 nucleotides (see the definition here above).

Advantageously, the plurality of the DNA constructs shares the same promoter sequence (for the transcription in a RNA molecule). Preferably, the promoter sequence is a bacteriophage RNA polymerase promoter. More preferably, the promoter sequence belongs to a group of promoter sequences comprising a promoter sequence of T7 RNA polymerase (SEQ ID NO:1), of SP6 RNA polymerase, of T3 RNA polymerase, of Syn5 RNA polymerase, of KP34 RNA polymerase. Favorably, the promoter sequence according to the present invention is the promoter sequence of T7 RNA polymerase. Detailed explanations about the DNA construct as well as the DNA sequences of the DNA construct can be found in EP21196390.5 filed on September 13, 2021.

Preferably, the transcription factor recognizing the shared promoter sequence is added during the transcription (transcribing) step.

According to the present invention, the plurality of synthetic mRNA constructs in the pool is obtainable (obtained) by a process for the production of the plurality of synthetic DNA constructs comprising the steps of performing the synthesis of :
(i) a first single stranded DNA molecule comprising a promoter for the transcription in RNA, a segment for its translation and a targeting sequence for endosomial addressing fused in-frame with a tumor neoantigen oran epitope from an infectious agent and a first portion of a genetic element encoding a sequence for the loading of the encoded tumor neoantigen or the epitope from an infectious agent for presentation to T lymphocytes, and
(ii) a second single stranded DNA molecule encoding a second portion of a genetic element encoding a sequence for the loading of the encoded tumor neoantigen or the epitope from an infectious agent for presentation to T lymphocytes, wherein the second single stranded DNA molecule is antisense and wherein the said first and second portions form the complete sequence for the loading of the tumor neoantigen or the epitope from an infectious agent for presentation to T lymphocytes and wherein the said first and second portions present an overlap (of at least 15 consecutive nucleotides) allowing a specific base pairing,

the said process further comprising the step of hybridizing the DNA molecule under (i) with the DNA molecule under (ii) and of elongating the two molecules so as to generate a double stranded DNA molecule encoding a promoter a translational initiator, a targeting sequence for endosomial addressing fused in-frame with the tumor neoantigen or the epitope from an infectious agent and a genetic element encoding a sequence for the loading of the encoded tumor neoantigen or the epitope from an infectious agent for presentation to T lymphocytes, then of transcribing in vitro the plurality of the DNA molecules so as to form a plurality of mRNA constructs.

According to the present invention, the synthesis of the first single stranded DNA molecule and possibly of the second single stranded DNA molecule is (are) preferably synthesis using a silicon-based DNA writing technology. Alternatively, the second single-stranded molecule is a 'master stock', that has been synthetized once, is enzymatically amplified, and stored, to increase the efficiency of the overall process, since only one molecule needs to undergo a chemical synthesis.

Preferably, the process according to the present invention further comprises the preliminary step of optimizing the sequences of the plurality of DNA, preferably by selecting more abundant codons, and/or of avoiding detrimental secondary structures. Detrimental secondary structures can be predicted using available *in silico* tools, thus, when possible, avoided.

Preferably, pseudouridine is added during the transcription step into RNA.

Preferably, pseudouridin is added, further to uridine or replacing all the uridine, during the transcription step into RNA. Instead, or in addition to pseudouridine, and/or of N1- Methylpseudouridine, 15-methyluridine (m5U), and/or 2-thiouridine (s2U) can be incorporated in the mRNA.

Preferably, the RNA sequence of each mRNA of the plurality of mRNAs is enriched in pseudouridine, possibly by using codon degeneracy, while maintaining the sequence of the corresponding encoded peptide.

Preferably, as mentioned, the RNA sequence comprises a 5' Cap element, comprising two nucleotides separated (linked in 5'-3') by a triphosphate stretch. The two nucleotides can be natural, or modified, for instance by methylation of the -OH residue at the 2' position (2-O-Me nucleotides). The 5'Cap element can comprise additional groups, including additional nucleotides, provided that two nucleotides are separated (linked in 5'-3') by a triphosphate group, instead of a monophosphate.

Preferably, the plurality of different DNA molecules is of at least 40 different DNA molecules encoding at least 40 different epitopes, preferably at least 50, or at least 60, 80, 100 or even 120 different epitopes.

Preferably, the process according to the present invention further comprises the step of amplifying the plurality of synthetic DNA constructs to generate an amplified pool of DNA constructs, wherein the amplified pool of DNA constructs is used as template for in vitro transcribing step.

Preferably, the process for producing a mRNA vaccine further comprises, after the in vitro transcribing step, a step of checking the quality and/or the quantity of each mRNA of the plurality of mRNAs. Preferably, the quality and/or the quantity of each mRNA of the plurality of mRNAs is performed by sequencing the plurality of mRNAs.

Preferably, the process for producing a mRNA vaccine also further comprises a step of formulating the plurality of mRNAs, optionally with one or more adjuvants, into a vector administrable to a patient. However, adjuvants are not required for such RNA vaccination, especially when uridine residues are less present, or totally replaced by pseudouridine.

The present invention also refers to a mRNA cancer vaccine for a patient having a tumor comprising a plurality of mRNAs encoding a plurality of neoantigens, wherein said plurality of neoantigens is deduced from the tumor, wherein said plurality of mRNAs encodes for at least 1% of all tumor neoantigens identified in the tumor, preferably for at least 2%, more preferably at least 5%, even more preferably at least 10%, 20%, 30%, 40%, 50%, 60%, 70%, 80%, 90% of all tumor neoantigens identified in the tumor.

Preferably, the mRNA vaccine according to the present invention, as above-described, further comprises (i) a segment encoding a sequence for stabilizing and/or targeting and/or trafficking the tumor neoantigen, and/or (ii) a segment encoding a sequence for addressing the encoded tumor neoantigen to MHC molecules, and/or (iii) a segment encoding a translation enhancer, and/or (iv) a 3'-UTR and/or (v) a 3' Poly A tail segment(s) and/or (vi) a 5'-UTR.

Preferably, the RNA sequences comprise a 5' Cap element, comprising two nucleotides separated (linked in 5'-3') by a triphosphate stretch. The two nucleotides can be natural, or modified, for instance by methylation of the -OH residue at the 2' position (2-O-Me nucleotides). The 5'Cap element can comprise additional groups, including additional nucleotides, provided that two nucleotides are separated (linked in 5'-3') by a triphosphate group, instead of a monophosphate.

Preferably, the plurality of mRNAs encodes for at least 2%, preferably at least 5%, more preferably at least 10%, 20%, 30%, 40%, 50%, 60%, 70%, 80%, 90% of all tumor neoantigens identified in the tumor.

Advantageously, the plurality of mRNAs is incorporated in one or more lipid nanoparticles. Preferably, the plurality of mRNAs is complexed with positively-charged lipids. Preferably, the plurality of mRNAs and the positively-charged lipids are mixed in microfluidic chips.

Optionally, one or more type 1 adjuvants is (are) incorporated with the plurality of mRNAs to form the mRNA cancer vaccine, wherein the one or more adjuvants is preferably selected from a group of adjuvants of type 1 having amorphous aluminum hydroxyphosphate sulfate (AAHS), aluminum hydroxide, aluminum phosphate,potassium aluminum sulfate (Alum), or Monophosphoryl lipid A. However, such adjuvants are not required, especially when pseudouridin has been incorporated in the mRNA vaccine.

Optionally, the plurality of mRNAs further comprises adjuvants of type 2 in the form of DNA or of mRNA such adjuvant of type 2 encoding for constitutively active TLR4 (caTLR4) and/or CD70 and/or CD40L and/or interferon-gamma and/or decoy interleukin receptors, i.e. Interleukin 1 receptor type II. Advantageously, the adjuvant DNA or mRNAs of type 2 promotes the stimulation, when incorporated into the mRNA vaccine, of dendritic cells of the patient and increases the efficacy of presentation of the plurality of neoepitopes to T cells of the patient. Such adjuvants are useful especially for the *ex vivo* maturation of dendritic cells of the patient.

Preferably, the plurality of mRNAs further comprises adjuvants of type 3 in the form of DNA or of mRNA such adjuvant of type 3 encoding for a RNA-dependent RNA polymerase (RdRp), such as DNA or RNA encoding for the nonstructural proteins of the alphavirus nsP1-4. Indeed, it is useful that the plurality of mRNAs of the mRNA vaccine is able of trans-amplification, such as the trans-amplification based on the encoded RdRp. Such RNA trans-amplification permits to reduce the dose of mRNA vaccine administrated to the patient.

Preferably, at least 25%, preferably at least 30%, 40%, 50%, 60%, 70%, 80%, 90%, 95% or even 100% of uridine present in the RNA sequence of each mRNA of the plurality of mRNAs is replaced by pseudouridine or N1-methylpseudouridine or 15-methyluridine or 2-thiouridine. More preferably, at least 25%, preferably at least 30%, 40%, 50%, 60%, 70%, 80%, 90%, 95% or even 100% of uridine present in the RNA sequence of each mRNA of the plurality of mRNAs is replaced by pseudouridine.

The present invention also refers to ex *vivo* method to select neoantigens to a patient comprising the step of submitting a blood sample from a patient having been administered with the mRNA cancer vaccine according to the present invention or with the RNA vaccine obtainable by the process according to the present invention to the corresponding synthetic peptide neoepitopes, or to one or several truncated version thereof, and of identifying which synthetic peptide neoepitopes triggers an immune response.

Indeed, this method to select neoantigens of a tumor of a patient comprising the step of submitting an ex *vivo* blood sample of this patient, having previously been given the plurality of the tumor neoantigens as described here above, and on testing against which neoepitopes does the said blood sample react.

This method allows to rapidly generate a more refined pool of neoantigens for treating this patient.

In practice, the peptides corresponding to the identified neoantigens are synthetized, then formulated in an aqueous solution in different compartments, and one peptide is allowed to react in a given compartment with the *ex vivo* sample. For neoantigens being too long peptides or predicted to have a too poor water solubility, truncated peptides can be generated having a sequence deduced from this neoantigen, for instance a peptide starting with the N-terminus extremity of the neoantigen, or with an amino acid downstream (e.g. 1, 2, 3, 4 or 5 amino acids from the N-terminal end) to that N-terminus extremity and finishing at the first identified mutation, or after 1, 2, 3, 4, 5 amino acid from that identified mutation. Alternatively or, preferably, in addition, a second overlapping peptide is synthetized, where the first identified mutation is flanked by 3, 4, 5, 6, 7, 8, 9 amino acids upstream the first mutation and flanked by 3, 4, 5, 6, 7, 8, 9 amino acids downstream the first mutation. Alternatively, or, preferably, in addition, a (second or third) overlapping peptide starting from the first identified mutation, or 1, 2, 3, 4, 5 amino acids upstream this first identified mutation, and terminating at the C-terminal end of the neoantigen, or 1, 2, 3, 4, 5 amino acids upstream of this C-terminal end of the neoantigen is synthetized as well. These (two or three) overlapping peptides can be placed in the same compartment for testing, or in two or three different compartments of the mRNA cancer vaccine according to the present invention or obtainable by the process according to the present invention for in vitro identification of a series of neoantigens of the tumor of the patient.

The present invention also refers to the neoantigens selected according to the ex vivo method of the present invention for use in vaccination of a patient affected by a tumor.

The present invention also refers to a method for expanding in vitro cytotoxic T cells specific against a tumor affecting a patient comprising the step of conditioning a blood sample from the said patient with the selected neoantigens of the present invention.

### Examples.-

### Example 1: Preparation of 97 mRNAs corresponding to neoepitopes identified in a lung tumor of a patient

The inventors have isolated the DNA and the RNA from a sample of a lung tumor of a patient. The inventors also isolated the DNA from normal cells (peripheral blood mononuclear cells, PBMC) of the patient. Then, the following steps were performed:
- sequencing the DNA from the tumor, the DNA from PBMC cells and the RNA,
- identifying the DNA variants of the tumor encoding for neoantigens of the tumor by comparing the DNA sequenced data from the DNA of the tumor and the DNA from PBMC cells and by identifying the open reading frames of the tumor having a DNA variant leading to the detection of a mRNA transcript.
- identifying the neoantigens of the tumor, a plurality of 97 different neoantigens has been identified,
- generating 97 double-stranded DNA templates comprising each a DNA sequence encoding for 1 different neoepitope having an amino acid sequence of 27 amino acids, wherein each neoepitope sequence is a part of the sequence of one neoantigen,
- in vitro transcribing the 97 double-stranded DNA templates to produce the 97 mRNAs.

### Example 2: Composition of a mRNA vaccine according to the present invention

The inventors developed a mRNA vaccine comprising:
- 97 different mRNAs encoding each a neoepitope of 27 amino acids identified from the lung tumor of the patient. The total amount of these 97 different mRNAs is of 1000 µg per dose of mRNA vaccine.
- positively-charged lipids.

### Example 3: Quality and quantity of the 97 different mRNAs included into the mRNA vaccine

Before incorporating the plurality of mRNAs (see 97 different mRNAs of the Example 1) into the mRNA vaccine composition (see Example 2), the inventors checked the quality and quantity of each mRNA of the plurality of mRNAs by performing an RNA sequencing. To do so, the inventors prepared double strand cDNA synthesis, using reverse transcriptase, and performed Illumina adapters ligation and cDNA library amplification by PCR. RNA Sequencing is then carried out in paired-end 100b mode with an high throughput equipment, here the Illumina NovaSeq 6000.

### Example 4: Use of the mRNA vaccine as a diagnostic tool to produce a more efficient personalized mRNA vaccine by detection of T cells activation

The inventors used the mRNA vaccine of Example 2 as a diagnostic tool to identify amongst the 97 different mRNAs encoding each a different neoepitope, the neoepitopes that stimulate T cells of the patient.

To do so, the mRNA vaccine according to the Example 2 was administrated to the patient having a lung tumor. After 10 days, blood of the patient is collected, wherein peripheral blood mononuclear cells and T cells were isolated. T cells from PBMC of the blood were isolated by anti-CD3 magnetic beads (Milteny). In parallel, 3*97 peptides corresponding to the neoepitope amino acid sequences of the 97 different mRNAs were synthetized in an amount of 1 to 4 microgram of each peptide; in every case, one peptide starting from the N-terminal end of the neoantigen and finishing three amino acids after the first identified difference (mutation); the second peptide starting 7 amino acids before the first identified difference (mutation) and finishing 7 amino acids after it, and the third peptide starting 3 amino acids ahead of the first difference (mutation) and finishing at the C terminal end of the neoepitope. Each peptide of the 3* 97 neoepitopes is tested by an ELISpot assay wherein T cells isolated from the blood of the patient are put in contact with each peptide (the three peptides derived from one neoepitope are present, combined, in one well). Then the inventors detect if T cells of the patient were activated by a given neoepitope (peptide) encoded by the mRNA vaccine by detecting the production of IFN-gamma in the well corresponding to the given neoepitope (peptide).

From the 97 neoantigens, the inventors identified 63 neoantigens that activate T cells of the patient.

### Example 5: Use of the mRNA vaccine as a tool to produce a second generation of mRNA vaccine by detection of CD8 cells activation

As in the Example 4, the mRNA vaccine according to the Example 2 was administrated to the patient having a lung tumor. After 10 days, a blood sample of the patient is collected, wherein peripheral blood mononuclear cells (PBMC) and T cells were isolated. T cells from PBMC of the blood were isolated by anti-CD3 magnetic beads (Milteny). From this isolated fraction of CD3+ cells (T cells), CD8+ and CD4+ cells were isolated by anti-CD8 and anti-CD4 magnetic beads, respectively. The fraction of CD3+ cells depleted from the CD8+ cells and CD4+ cells represents a fraction of antigen-presenting cells (APC). In parallel, 3* 97 peptides corresponding to the neoepitope amino acid sequences of the 97 different mRNAs were synthetized using the same approach as in Example 4, in an amount of 1 to 4 microgram of each peptide. Each peptide of the 97 neoantigens is tested by an ELISpot assay wherein T cells isolated from the blood of the patient is put in contact with each neoantigen (the three peptides derived from one neoantigen are present, combined, in one well). Then, the antigen-presenting cells (APC) fraction is electroporated with each peptide in the presence of the CD8+ cells. Then the production of IFN-gamma has been measured.

From the 97 neoantigens, the inventors identified 24 neoantigens that activate specifically CD8+ cells of the patient.

### Example 6: Use of the mRNA vaccine as a tool to produce a more efficient personalized mRNA vaccine by detection of CD107a.

The same steps as in Example 5 were performed. In addition, CD107a expression were measured in each well by using an anti-CD107a marker. CD107a is a marker of degranulation (indicator of functional cytotoxicity of CD8+ cells).

From the 24 neoantigens that activate specifically CD8+ cells of the patient (see Example 5), 7 neoantigens were identified to be functionally cytotoxic by measuring an expression of CD107a.

### Example 7: Optimized mRNA vaccine

2 months after the injection of the mRNA vaccine in the lung tumor of the patient (see Example 5), a 300 µg of the optimized mRNA vaccine comprising the 7 identified neoantigens were administrated to the patient.

### Example 8: Second generation cell-therapy

For some patients, the cell therapy approach is preferred; hence the inventors have taken the advantage of the above wide and refined system to produce *in vitro* T cells able to directly attack the tumor. To do so, 2 months after the injection of the mRNA vaccine in the lung tumor of the patient and the identification of the optimized neoepitopes as in examples 4-7, cell therapy of the tumor were performed by isolating the 7 CD8+ CD107a+ cell lines of the patient. To do so, DC cells of the patient have been maturated using the 7 optimized neoepitope administered as a mRNA vaccine, together with the administration by transfection of a plasmid encoding active TLR4 (caTLR4), CD70 and CD40L. Then the 7 CD8+ CD107a+ cell lines of the patient were expanded ex vivo before being reinjected into the patient having a lung tumor.

## Claims

1. A process for producing a RNA vaccine comprising a plurality of epitopes specifically deduced from a target comprising the steps of:
- obtaining a plurality of different synthetic DNA constructs encoding a plurality of different epitopes deduced from the said target and being in proximity of a sequence allowing the transcription of the said different epitopes encoded by the said different synthetic DNA constructs into a RNA sequence and of a sequence for the translation of the said transcribed RNA into a peptide by a eukaryotic cell,
and of
- transcribing *in vitro* the said plurality of synthetic DNAs into a corresponding plurality of RNAs,
wherein the said target is a peptide from an infectious agent, or cancer neoepitopes specifically identified in one patient as having at least one amino acid difference as compared with peptides from normal cells of the said patient.

2. The process of claim 1, wherein the DNA construct further comprises a plurality of sequences elements encoding a peptide sequence for endosomial targeting of the encoded peptide.

3. The process of claim 1 or 2, wherein the DNA construct further comprises a plurality of sequences elements encoding a peptide sequence for the loading of the encoded peptide for presentation to T lymphocytes and/or wherein the DNA construct further comprises at least one sequence element encoding a translation enhancer and/or wherein the DNA construct further comprises at least one sequence element encoding 3'-UTR and/or a 3' Poly A tail segment(s), preferably wherein the plurality of the different DNA constructs share the same plurality of sequences elements encoding a peptide sequence for endosomial targeting of the encoded peptide and/or the same plurality of sequences elements encoding a peptide sequence for the loading of the encoded peptide for presentation to T lymphocytes.

4. The process according to any one of the preceding claims, wherein the plurality of the DNA constructs shares the same promoter sequence.

5. The process of claim 4, wherein the transcription factor recognizing the shared promoter sequence is added during the transcription step.

6. The process according to any one of the preceding claims, wherein pseudouridine is added during the transcription step into RNA.

7. A process according to any one of the preceding claims, further comprising the preliminary step of optimizing the sequences of the plurality of DNA, preferably by selecting more abundant codons, and/or of avoiding detrimental secondary structures.

8. The process according to any one of the preceding claims, wherein the plurality of different DNA molecules is of at least 40 different DNA molecules encoding at least 40 different epitopes, preferably at least 50, or at least 60, 80, 100 or even 120 different epitopes.

9. The process according to any one of the preceding claims, further comprising the step of adding a 5' Cap element to the different epitopes, wherein the Cap element comprises two nucleotides separated by a triphosphate stretch.

10. A mRNA cancer vaccine for a patient having a tumor comprising a plurality of mRNAs encoding a plurality of neoantigens, wherein said plurality of neoantigens is deduced from the tumor, wherein said plurality of mRNAs encodes for at least 1 % of all tumor neoantigens identified in the tumor, preferably for at least 2%, more preferably at least 5%, even more preferably at least 10%, 20%, 30%, 40%, 50%, 60%, 70%, 80%, 90% of all tumor neoantigens identified in the tumor.

11. The mRNA cancer vaccine according to claim 10, wherein at least 25%, preferably at least 30%, 40%, 50%, 60%, 70%, 80%, 90%, 95% or even 100% of uridine present in the RNA sequence of each mRNA of the plurality of mRNAs is replaced by pseudouridine or N1-methylpseudouridine or 15-methyluridine or 2-thiouridine.

12. *An ex vivo* method to select neoantigens to a patient comprising the step of submitting a blood sample from a patient having been administered with the mRNA cancer vaccine according to the preceding claims 10 or 11 or with the RNA vaccine obtainable by the process according to the preceding claims 1 to 9 to the corresponding synthetic peptide neoepitopes, or to one or several truncated versions thereof, and of identifying which synthetic peptide neoepitopes triggers an immune response.

13. The method of claim 12, wherein the immune response is a cytotoxic immune response.

14. The neoantigens selected according to claim 12 or claim 13 for use in vaccination of a patient affected by a tumor.

15. A method for expanding in vitro cytotoxic T cells specific against a tumor affecting a patient comprising the step of conditioning a blood sample from the said patient with the selected neoantigens of claim 12 or 13.
